# EUROPÄISCHE PATENTSCHRIFT

(11) **EP 0 988 324 B1**
(45) Veröffentlichungstag und Bekanntmachung des Hinweises auf die Patenterteilung: **04.02.2004**
(21) Anmeldenummer: 98922576.8
(22) Anmeldetag: 11.06.1998
(51) Int. Cl.: C08B 37/00, A23L 1/0522, A23L 1/0526, A61K 31/715, A61K 47/36, A23K 1/16, C09D 105/00, C06B 23/00

(54) **VERFAHREN ZUR HERSTELLUNG VON REINEM GUARKERNMEHL**
METHOD FOR MANUFACTURING PURE GUAR MEAL
PROCEDE POUR LA PREPARATION DE FLEUR DE FARINE DE GUAR PURE

(30) Priorität: 12.06.1997 EP 97810369
(43) Veröffentlichungstag der Anmeldung: 29.03.2000
(73) Patentinhaber: Meyhall AG, 8280 Kreuzlingen (CH)
(72) Erfinder: WIELINGA, Willem, Cor, CH-8274 Tägerwilen (CH); RICCA, Jean-Marc, F-75012 Paris (FR)
(74) Vertreter: Groner, Manfred
(86) Internationale Anmeldenummer: PCT/CH1998/000256
(87) Internationale Veröffentlichungsnummer: WO 1998/056828

(56) Entgegenhaltungen:
- EP-A- 0 130 946
- EP-A- 0 246 854
- EP-A- 0 432 951
- EP-A- 0 465 992
- EP-A- 0 686 643
- WO-A-97/11974
- DATABASE WPI Section Ch, Week 9246 Derwent Publications Ltd., London, GB; Class D21, AN 92-375610 XP002073157 & JP 04 273 811 A (KAWAKEN FINE CHEM CO LTD)

## Beschreibung

Gegenstand der vorliegenden Erfindung ist ein Verfahren zur Herstellung von Guarkernmehl, das, wenn es in Wasser gelöst vorliegt, eine transparente Lösung unterschiedlicher, d.h. geringer bis sehr hoher Viskosität ergibt, wobei das Verfahren trotz extensiver Reinigung gute Ausbeuten des reinen Mehls liefert. Transparente, hochviskose Lösungen aus reinem Guarkernmehl sind vor allem in der Nahrungsmittelindustrie von grosser Bedeutung.

Guarkernmehl findet als Verdickungsmittel im Textilund Sprengstoffsektor, als Bindemittel in der Papierindustrie, als Flockungsmittel bei der Erzgewinnung und Hilfsmittel bei der Erdgas- und Erdölförderung, im pharmazeutischen und kosmetischen Bereich, und als Verdickungsmittel, Emulgator und Co-Stabilisator im Nahrungsmittelbereich und der Nahrungsmitteltechnologie Verwendung.

In der Pharmazie wird niedrigviskoses Guarkernmehl zur Sprüheinbettung zum Beispiel von Vitaminen verwendet, um deren Lagerstabilität zu erhöhen. Darüberhinaus garantiert die Verwendung von Guarkernmehl in Sprays eine nahezu monomolekulare Verteilung der Wirkstoffe und eine dadurch verbesserte, gleichmässige Resorption, was im Fall von Asthmamitteln und diversen Antiallergika wünschenswert ist. Durch den ausserordentlich geringen Proteingehalt des reinen Guarkernmehls besteht keine Gefahr für die Ausbildung allergischer Reaktion auf ein diese Substanz enthaltendes Medikament. Weitere Anwendungen auf diesem Gebiet sind die Formulierung von Retardtabletten und als Mittel zur Senkung des Cholesterinspiegels. Im medizinischen Bereich wird stark visköses Guarkernmehl auch als Stabilisator in Kontrastmitteln verwendet.

Guarkernmehl hat sich unter anderem auch als ideales diätetisches Mittel erwiesen, da seine Bausteine, die sogenannten Galaktomannane, von den menschlichen Magenund Dünndarmenzymen nicht angegriffen werden. Dies ist insofern zu erwarten, da im resorbierenden Teil des menschlichen Verdauungssystem weder β-Mannanasen noch β-Mannosidasen oder α-Galaktosidasen vorhanden sind, die zur Aufspaltung dieser Bausteine notwendig wären. Da die Bausteine des Guarkernmehls nicht in den menschlichen Stoffwechsel eingehen, ist das Guarkernmehl in keiner Weise als Kalorienträger oder -lieferant anzusehen. Da sich das Guarkernmehl aus völlig neutralen Polysacchariden, d.h. genauer aus Galaktomannanen, zusammensetzt, die weder Uronsäure noch andere ionogene Gruppen aufweisen, stellen sie in physiologischer Hinsicht völlig unbedenkliches Material dar.

Ein weiterer Vorteil im Hinblick auf seine Verwendung als Nahrungsmittelzusatzstoff ist seine völlige Geschmacksneutralität. Es findet Verwendung in kalorienoder fettreduzierten Nahrungsmitteln oder Getränken, die von dem Konsumenten häufig als "dünn" empfunden werden. Die Zugabe von reinem Guarkernmehl zu diesen Produkten verleiht ihnen eine "sahnigere" Konsistenz. Bei der Herstellung von Fruchtsäften wird Guarkernmehl verwendet, um die Fruchtpulpe gleichmässig zu resuspendieren, in Puddings und Cremes dient es als Verdikkungsmittel, in Eiscremes, Milchshakes, Mousse und ähnlichen Produkten als Stabilisator.

Mit herkömmlichen Guarkernmehlpräparaten war nur eine geringe molekulare Wechselwirkung mit dem Biopolymer Xanthan zu verzeichnen. Beim Mischen dieser beiden Kolloide trat zwar eine synergistische Viskositätserhöhung auf, eine spezifische Gelbildung wie im Fall von Carubin, dem Johannisbrotkernmehl und Xanthan trat jedoch nicht auf. Wenn man Mischungen in einem Verhältnis von 1:1 aus dem Guarkernmehl erhalten gemäss der Erfindung und Xanthan gemeinsam erhitzt und bei 4° C (Kühlschranktemperaturen) abkühlen lässt, bildet sich ein weiches Gel. Ein Vorteil dieser Kombination aus Guarkernmehl und Xanthan besteht darin, dass das Gel aus diesen beiden Komponenten bei Körpertemperatur schmilzt und sich daher hervorragend zur Herstellung von geleeartigen Lebensmitteln, als Trägersubstanz bei der Verabreichung von Medikamenten in Zäpfchenform und ähnlichem eignet. Guarkernmehl und Xanthan werden darüber hinaus gemeinsam als Costabilisatoren bei der Herstellung von Salatdressings verwendet, da diese Kombination, im Gegensatz zu Guarkernmehl, wenn es allein verwendet wird, säureresistenter ist.

Guarkernmehl wird aus dem Endosperm der Guarbohne (*Cyamopsis tetragonobolus*) gewonnen. Guarkernmehl besteht weitgehend aus Galactomannanen, d.h. aus Polysacchariden, deren Hauptkette in 1 → 4 Richtung durch β-glykosidische Bindungen verknüpft ist, und setzt sich aus Mannose zusammen, die teilweise über primäre OH-Gruppen mit Galaktose verbunden ist. Das Verhältnis von unsubstituierter zu mit Galaktose substituierter Mannose beträgt ca. 2:1, wobei die substituierten Einheiten nicht streng alternierend, sondern in Zweier- oder Dreiergruppen in den Polygalactomannanmolekülen angeordnet sind. Die Guar-Galactomannane bilden schon in geringen Konzentrationen mit Wasser hochviskose Lösungen. 1 gewichtsprozentige Lösungen von handelsüblichem Guarkernmehl in Wasser ergeben Viskositäten von ca. 3'000 bis 6'000 mPa.s.

Guar-Galaktomannane sind aufgrund chemischer und physicochemischer Unterschiede in kaltwasserlösliche, heisswasserlösliche und unlösliche Galaktomannane aufgeteilt worden.

Zur Gewinnung und Reinigung des Guarkernmehls wird die Guar-Saat mechanisch behandelt, wobei ungefähr 35 Teile unreiner Guar-Endospermhälften und ungefähr 60 Teile Guarmehl erhalten werden. Das Guarmehl besteht im wesentlichen aus dem Keim der Saat, der abgeschürften Bohnenschale und kleinen Endospermteilen. Das Endosperm umhüllt den Keim vollständig und wird seinerseits von der Samenschale umschlossen. An den Kontaktstellen zwischen Endosperm und Samenschale befindet sich eine proteinreiche, aleuronähnliche Zellschicht, deren Zellen eng mit dem Endosperm verzahnt sind.

Die unreinen Endospermhälften können weiter mechanisch gereinigt werden und liefern Splits von unterschiedlicher Qualität hinsichtlich ihres Proteingehalts, ihrer durch Säure nicht hydrolisierbaren Bestandteile (A.I.R.) sowie des Schalengehalts. Die in Fachkreisen übliche Bezeichnung "Split" ist dem Begriff "Endospermhälften" gleichzusetzen.

Obwohl Guarkernmehl als Verdickungsmittel bereits eine breite Anwendung findet, ist es erwünscht, seinen Reinheitsgrad und damit verbunden seine physikalischen und physiologischen Eigenschaften zu verbessern. Insbesondere für seine Anwendung im Nahrungsmittelbereich ist die Reinheit des Guarkernmehls von grosser Wichtigkeit. Wünschenswert ist ebenfalls eine bessere Ausnutzung der neutralen, nicht-ionogenen Hauptbestandteile des Endosperms, so dass diese vermehrt anstelle von in Wasser klar löslichen Cellulosederivaten oder anderen Polysacchariden oder in Wasser klar löslichen synthetischen Polymeren in den entsprechenden Industriezweigen angewendet werden können.

Werden die zu Mehl verarbeiteten, zur Zeit auf dem Markt erhältlichen, aus reinem Guarkernmehl bestehenden Produkte in Wasser bei 25° C oder 86 bis 89° C 10 Minuten gelöst, werden trübe Lösungen erhalten. Wird das unlösliche Material dieser Lösungen mit hohen Zentrifugalkräften (>35.000 x g) ausgeschleudert, stellt sich heraus, dass 23 - 35 % des Guarkernmehls aus ausgeschleudertem Material besteht.

Mikroskopische Untersuchungen haben gezeigt, dass sich das ausgeschleuderte Material hauptsächlich aus Schalenfragmenten, Proteinkörpern, unlöslichen peripheren Zellen, intakten, nicht aufgeschlossenen Zellen des inneren Endosperms und anderen Samen- beziehungsweise Splitverunreinigungen zusammensetzt. Eine bekannte chemische Derivatisierung des Guarkernmehls durch Verätherung, Karboxymethylierung, Hydroxypropylierung, deren Kombination sowie Kationisierung ermöglicht die Herstellung von Produkten mit signifikant verbessertem Lösungsverhalten in Wasser und damit einhergehend höherer Transparenz der Lösungen.

Eines der bisher angewendeten Verfahren zur Gewinnung von reinem Guarkernmehl verwendet chlorierte Lösungsmittel, wie zum Beispiel Trichlorethylen (siehe EP 0 130 946, Meyhall Chemical AG). Die Suspension wurde durch einfaches Stehenlassen oder Zentrifugieren fraktioniert, wobei sich eine proteinreiche Fraktion (flottierende Fraktion) ausbildete und sich eine proteinarme Fraktion (Sinkfraktion) abschied.

Es konnte gezeigt werden, dass die obere flottierende Fraktion aus zu Mehl verarbeitetem Endosperm wie Guar CSA 200/50 bis zu 25 % Proteine enthalten kann und die Sinkfraktion, die 75 % des reinen Mehls ausmacht, etwa 1,5 bis 1,6 % Protein enthält. Die Sinkfraktion ist zum Beispiel zur Herstellung von kationischen Derivaten geeignet, die nach ihrer Lösung klare wässrige Lösungen ergeben. Nachteil dieses Verfahrens ist, dass feingemahlene Schalenfragmente ebenfalls in der Sinkfraktion vorgefunden werden.
Ein weiterer Nachteil ist die Verwendung von halogenierten Lösungsmitteln, da ein spezifisches Gewicht von 1,47 bis 1,48 kg/l erforderlich ist. Proteine besitzen eine Dichte von 1,3 kg/l und die Galactomannane eine Dichte von 1,5 bis 1,55 kg/l, je nach Feuchtigkeitsgehalt. Das mit dem beschriebenen Verfahren hergestellte Guarkernmehl ist nur für technische Anwendungen geeignet, im Nahrungsmittelbereich ist dieses Guarkernmehl nicht anwendbar, da Reste des verwendeten halogenierten Lösungsmittels, 10 ppb wurden in mit Äthanol extrahierten Fraktionen nachgewiesen, in dem Endprodukt verbleiben. Halogenierte Lösungsmittel sind in unterschiedlichem Mass toxisch und ätzend und besitzen häufig allergisierende Eigenschaften. Auch aus Umweltgründen sollte von diesem Verfahren abgesehen werden.

Ein weiteres Verfahren zur Herstellung von reinem Guarkernmehl wurde bereits 1969 vorgeschlagen. Es bestand aus einer Alkalibehandlung von vorgequollenen Splits bei erhöhten Temperaturen, wobei 100 Teile Alkali von 100 Teilen SPS absorbiert wurden. Die grosse Menge Alkali, d.h. NaOH, musste ausgewaschen werden. Dies wurde mit kaltem Wasser in einem Verhältnis von einem Teil SPS (Single Purified Splits) zu 80 Teilen H₂O und in einem Entwässerungsschritt mit Isopropanol (IPA) durchgeführt, in dem gleichzeitig die restliche NaOH der gereinigten Splits durch Essigsäure neutralisiert wurde.

Nach dem Mahlen wurde ein reines Guarkernmehl von hoher Qualität in einer Ausbeute von 60-70 %, auf dem Rohmaterial SPS (einfach gereinigte Splits) basierend, gewonnen. 1969 wurde dieses Verfahren von Stein, Hall & Co, Long Island City, New York, bis zur-Industriereife weiterentwickelt. Das derzeitige Waschverfahren des carboxymethylierten, hydroxypropylierten oder kationisierten Guarkernmehls (Guarether) oder deren Kombinationen mit Wasser beruht auf diesem Verfahren. Der Zweck dieses Reinigungsverfahrens von Guar-Derivaten ist es, Schalenfragmente und periphere Zellschichten zu entfernen, sowie Nebenprodukte der verschiedenen Verätherungsreaktionen (Hydroxypropylierung, Carboxymethylierung und Kationisierung und/oder ihre Kombinationen) zu entfernen.

Ein anderes bekanntes Verfahren zur Herstellung von reinem Guarkernmehl ist die Behandlung der Guar-Splits mit Säure. Dieses Verfahren liefert ein Produkt von ausgezeichneter Qualität, d.h. das resultierende Material ergibt, in Wasser gelöst, Lösungen von grosser Klarheit bei gleichzeitig hoher Viskosität. Ein Nachteil dieses Verfahrens besteht jedoch in der relativ aufwendigen Verfahrensweise mit mehrmaligen Wasch- und Neutralisationsschritten. Darüber hinaus werden für eine Säurebehandlung spezielle Apparaturen benötigt, die das Verfahren sehr kostenintensiv machen.

Trotz der oben beschriebenen extensiven Reinigungsverfahren ist es bislang nicht auf ökonomische und umweltfreundliche Weise gelungen, reines, nichtderivatisiertes Guarkernmehl zu erhalten, das eine klare, wässrige Lösung mit hoher Viskosität bei gleichzeitig guten Ausbeuten ergibt.

Die Nachteile der bisherigen Verfahrensweisen zur Reinigung und Gewinnung von reinem Guarkernmehl sind:
1. grosse Verluste wertvoller Endospermteile bei der mechanischen Reinigung und dadurch geringe Ausbeuten an reinem Guarkernmehl in Bezug auf das Ausgangsmaterial;
2. Schalenfragmente, die sich noch immer an den verschiedenen Splitqualitäten befinden und in einem grossen Ausmass die Funktionalität der modifizierten Endprodukte stören;
3. periphere, proteinreiche Zellen der Aleuronschicht, die in Wasser kaum quellen und ebenfalls die Funktionalität des Endprodukts negativ beeinflussen;
4. Vorhandensein anderer Verunreinigungen der Guar-Samen, wie Holzpartikel, die nicht vorhanden sein dürfen;
5. hohe Umweltbelastung

Es war daher dringend erwünscht, ein Verfahren zur Herstellung von reinem Guarkernmehl zu entwickeln, das die oben genannten Nachteile beseitigt und reines Guarkernmehl in guten Ausbeuten liefert, das nach seiner Dispersion in Wasser eine klare, hochviskose Lösung ergibt, die vor allem zum Beispiel in der Nahrungsmittelindustrie, pharmazeutische, Farben- und Streichmittelindustrie, sowie bei der Ölgewinnung Anwendung findet.

Ziel der vorliegenden Erfindung ist es, die obengenannten Anforderungen zu erfüllen, d.h. durch ein neues Herstellungsverfahren gute Ausbeuten an reinem, insbesondere für die Nahrungsmittelindustrie geeignetem Guarkernmehl zu erhalten, das niedrig bis hochviskose, ,klare wässrige Lösungen ergibt.

Das erfindungsgemässe Verfahren zur Herstellung von reinem Guarkernmehl ist in Patentanspruch 1 definiert und umfasst die folgenden Stufen:
(a) Behandeln der Guar-Splits mit einer Base in Anwesenheit von geringen Mengen Wasserstoffperoxid
(b) teilweise Neutralisation der Alkali-Splits mit einer Säure
(c) mechanisches Entfernen der peripheren Zellen
(d) gegebenenfalls zweimaliges Waschen mit Wasser
(e) Behandeln der Splits mit einer wässrigen Alkohollösung

Eine erste Voraussetzung zur Gewinnung reinen Guarkernmehls ist die Verbesserung des Ausgangsmaterials, der sogenannten Splits. Die mit Schale bedeckten Splits machen bis zu 42,5 Gewichtsprozent der Saat aus. Die überlappenden Schalen-Endosperm-Teile, die 13,5 Gewichtsprozent der Saat betragen, sind im wesentlichen in Wasser unlöslich. Der Keimling der Saat umfasst die restlichen 44 %. Diese Mengenangaben zeigen, dass die theoretische Ausbeute an für die Erfindung nutzbaren Splits ohne Schale und ohne überlappende Teile 32 % beträgt.

Das erfindungsgemäss erhaltene reine Guarkernmehl wird am vorteilhaftesten aus Splits hergestellt, die einen Proteingehalt von 4,2 % und einen A.I.R.-Anteil von 1,8 % haben.

Solche Splits können nach einer alkalischen Behandlung unter Verwendung von 10 bis 40 %iger, vorzugsweise 33 % Natronlauge bei Raumtemperaturen, vorzugsweise jedoch bei erhöhten Temperaturen hergestellt werden.

Der Unterschied dieses neuen gegenüber den bisher bekannten Verfahren, die ebenfalls eine Alkalibehandlung des Ausgangsmaterials einschliessen, besteht in der Zugabe von geringen Konzentrationen Wasserstoffperoxid während der Alkalibehandlung. Dieses chemische "Peeling" liefert Splits, von denen die Hüllzellen mit mechanischen Mitteln sehr leicht und nahezu vollständig entfernt werden können. Durch die Behandlung der Splits mit beispielsweise 8-10 Teilen 33 %iger Natronlauge und 1,1 Teilen 35 %igem Wasserstoffperoxid werden die peripheren Zellen der Splits 'attackiert und lassen sich durch mechanischen Abrieb entfernen. Die Polysaccharide der unter den peripheren Zellen befindlichen Zellschichten werden nicht oxidiert, da die Konzentration des eingesetzten Wasserstoffperoxids zu gering ist.

Die auf diese Weise gereinigten Splits, die wahlweise mit Wasser gewaschen oder auch ungewaschen weiterverarbeitet werden, können für die Zwecke der Erfindung noch signifikant verbessert werden, indem Phospholipide und andere "nicht polare" Substanzen ausgewaschen werden. Dies hat eine grössere Klarheit des in Wasser gelösten Produkts zur Folge und wird durch Behandlung des gereinigten Produkts mit einer wässrigen Alkohollösung, vorzugsweise wässriges Isopropanol (IPA), bei erhöhten Temperaturen erreicht. Bei nicht mit Wasser gewaschenen Produkten ist ein Zusatz weiterer Lauge vor der Behandlung mit Isopropanol nicht nötig, da das Produkt noch ca. 4-7 % NaOH enthält. Wurde das Produkt gewaschen, ist der Zusatz einer alkalischen Lösung erforderlich. Nach der Isopropanolbehandlung werden die alkalischen Splits mit Wasser gewaschen und bei einem gewünschten Feuchtigkeitsgehalt gemahlen.

Der Grad der Befeuchtung während des Mahlens beeinflusst die Eigenschaften des mehligen Endprodukts signifikant. Je höher der Feuchtigkeitsgehalt in einem technisch ausführbaren Mass ist, desto grösser ist die Menge der löslichen Polysaccharide, d.h. um so höher ist die Ausbeute an aktiven Galactomannanen. Dies kann durch die Vergrösserung des Zellvolumens, aufgrund des hohen Masses der Befeuchtung erklärt werden. Während des Mahlens werden die gequollenen Zellen durch eine definierte Öffnung oder Spalt gezwungen, wobei die Zellmembran reissen kann, vorausgesetzt, dass die gequollenen Partikel signifikant grösser sind als die Öffnungen (die Elastizität der Zellen spielt ebenfalls eine wichtige Rolle). Bei der Herstellung von Lösungen in Wasser werden die Galaktomannane aus den auf solche Weise zerstörten Zellen freigesetzt, was bei nicht zerstörten Zellen nicht der Fall ist. In diesen Fällen verbleiben die Galactomannane innerhalb der intakten Zellen und tragen nicht wirksam zu der Viskosität der Lösung bei.

Ein Feuchtigkeitsgehalt von ungefähr 82 %, vorzugsweise 72 bis 75 %, ist beim Mahlen aus praktischen und technischen Gründen annehmbar. Feuchtigkeitsgehalte niedriger als 72 % beim Mahlen beeinträchtigen die Qualität des Guarkernmehls. Ein höherer Gehalt als 82 % verursacht technische Probleme.

Ein grosser Vorteil der vorliegenden Erfindung liegt in der 25 %igen Rückgewinnung der abgeriebenen Periperiezellschichten, d.h. in einer extremen Verringerung der Umweltbelastung.

Ein weiterer Vorteil der vorliegenden Erfindung liegt in der Vereinfachung des Verfahrens. Nur noch wenige Schritte sind notwendig, um ein reines Produkt mit hoher Löslichkeit und Viskosität zu erhalten.

Ein weiterer Vorteil der vorliegenden Erfindung besteht in der Möglichkeit, Produkte für Lösungen mit zum Beispiel so niedrigen Viskositäten wie 35 mPa.s und solche mit bis zu 6000 bis 9.000 mPa.s bei 1 %iger Konzentration in Wasser bei 25° C gemessen herzustellen.

Ein weiterer Vorteil der Erfindung besteht darin, reine Guar-Produkte herzustellen, deren Proteingehalt so gering wie 0,2 bis 0,5 % ist.

Der grösste Vorteil der vorliegende Erfindung besteht jedoch darin, dass die bisher üblichen Reinigungsverfahren wesentlich vereinfacht und abgekürzt werden, wodurch die Herstellung des reinen Guarkernmehls wesentlich kostengünstiger wird.

Darüber hinaus ist das neue Verfahren ausgesprochen umweltschonend, da ca. 25 % der abgesiebenen Peripheriezellschichten zurückgewonnen werden können. Der Abrieb, der bei dem hier beschriebenen Verfahren entsteht, kann ausserdem als Verdickungsmittel im Textildruck verwendet werden. Dies bedeutet eine optimale Ausnutzung des Ausgangsmaterials.

Eine Derivatisierung der Galactomannane des Guarkernmehls ist für dessen Kaltwasserlöslichkeit von 'Bedeutung. Durch die Derivatisierung (z.B. Karboxymethylierung, Hydroxypropylierung, Kationisierung u. ä.) werden eine oder mehrere nicht-ionische, anionische oder kationische Gruppen hinzugefügt, wodurch die verätherten schwierig zugänglichen Galactomannane bereits bei 25°C in Lösung gehen. Die Derivatisierung findet üblicherweise im Anschluss an die Reinigung statt. Die Verwendung des derivatisierten Guarkernmehls ist allerdings in der Lebensmittelindustrie nicht erlaubt. Derivatisiertes, insbesondere kationenaktives, Guarkernmehl findet jedoch zum Beispiel in kosmetischen Produkten, wie Haarconditioner, Körperlotionen und ähnlichem Verwendung.

Zu den kationischen Guarkernmehlderivaten, die aus einem reinen, nach dem erfindungsgemäßen Verfahren erhaltenen Guarkernmehl hergestellt werden können, zählen ganz besonders das Hydroxypropyltrimethylammoniumchlorid-Guarkernmehl.

Diese können durch Veretherung eines reinen Guarkernmehls unter basischen Bedingungen und unter einer Inertgasatmosphäre mit 2,3-Epoxypropyltrimethylammoniumchlorid als kationisches Veretherungsmittel und anschließende Reinigung und Trennung erhalten werden.

Die Veretherung kann vorzugsweise in zwei Hauptschritten durchgeführt werden:
einem ersten Schritt, bei dem das kationische Veretherungsmittel in dem Guarkernmehl unter alkalischen Bedingungen und unter einer Inertgasatmosphäre bei einer Temperatur von ungefähr 20 bis ungefähr 55°C, Vorzugsweise ungefähr 30 bis ungefähr 50°C, diffundiert und anschließend in einer wäßrig-alkoholischen Lösung, insbesondere einer Wasser-Isopropanol-Lösung, die 25 bis 70 Gew.-% Isopropanol enthält, suspendiert wird,
und einem zweiten, eigentlichen Veretherungsschritt unter einer Inertgasatmosphäre bei einer Temperatur von ungefähr 50 bis ungefähr 70°C, besonders bevorzugt bei einer Temperatur von ungefähr 60 bis ungefähr 65°C.

Der erste Schritt (die Diffusion) kann insbesondere in Gegenwart von Natriumhydroxid durchgeführt werden, wobei auf 100 Teile Guarkernmehl ungefähr 1 bis 4 Gewichtsteile Natriumhydroxid entfallen; dieser Schritt kann ca. 15 bis 60 Minuten dauern.

Der zweite Schritt (die eigentliche Veretherung) kann ungefähr 45 bis 120 Minuten dauern.

Das entstandene kationische Produkt wird anschließend unter den gleichen Temperaturbedingungen wieder mit Luft in Kontakt gebracht, um das Produkt auf die gewünschte EndViskosität zu bringen. Das Produkt wird anschließend ein oder mehrmals durch Waschen mit einer wäßrig-alkoholischen Lösung, insbesondere Wasser/Isopropanol, gereinigt. Nachdem der pH mit einer Säure wie z.B. insbesondere Essigsäure eingestellt wurde, wird das kationische Guarkernmehl zum Beispiel durch Filtration oder Zentrifugation getrennt und dann getrocknet.

Die Menge an Veretherungsmittel, die verwendet werden kann, wird so gewählt, daß man ein kationisches Guarkernmehl mit einem Substitutionsgrad (SG) im Bereich von 0,01 bis 0,4 erhält. Der Substitutionsgrad (SG) kann definiert werden als die Molzahl an 2,3-Epoxypropyltrimethylammoniumchlorid, die pro Hexoseeinheit des Guarkernmehls zugegeben wird.

Die so erhaltenen Hydroxypropyltrimethylammoniumchlorid-Guarkernmehle können über ein mittleres Molekulargewicht von ungefähr 50 000 bis ungefähr 8 000 000 verfügen.

Die nach dem erfindungsgemäßen Verfahren erhaltenen kationischen Guarkernmehlderivate, insbesondere die Hydroxypropyltrimethylammoniumchlorid-Guarkernmehle, können insbesondere zur Herstellung von Kosmetikformulierungen verwendet werden.

Die Erfindung betrifft somit weiterhin die Verwendung eines durch Veretherung eines nach dem erfindungsgemäßen Verfahren erhaltenen Guarkernmehls hergestellten Hydroxypropyltrimethylammoniumchlorid-Guarkernmehls in klaren wäßrigen Kosmetikformulierungen, die zur Anwendung auf Haare und/oder Haut bestimmt sind und wieder ausoder abgespült werden sollen, als Konditionierungsmittel und/oder Ablagerungshilfsmittel ("deposition aid") für weitere Konditionierungsmittel bei der Verdünnung dieser Kosmetikformulierungen.

Unter "klarer wäßriger Kosmetikformulierung" versteht man jegliche Kosmetikformulierung, die mindestens 60 Gew.-% Wasser enthält und bei 600 Nanometern eine Durchlässigkeit von mindestens 92% aufweist.

Diese klaren Kosmetikformulierungen können insbesondere in der Form eines Konditionierungsshampoos, eines Duschgels oder einer Flüssigseife vorliegen.

Für eine vorteilhafte Ausführungsform der Erfindung weist dieses Hydroxypropyltrimethylammoniumchlorid-Guarkernmehl einen Substitutionsgrad (SG) von ungefähr 0,01 bis ungefähr 0,4, vorzugsweise von ungefähr 0,05 bis ungefähr 0, 25, und ein mittleres Molekulargewicht von ungefähr 50 000 bis ungefähr 3 . 10⁶ auf.

Es kann in einer Menge verwendet werden, die ungefähr 0,05 bis 0,5%, vorzugsweise ungefähr 0,1 bis 0,3%, des Gewichts dieser Kosmetikformulierungen entspricht.

Neben dem Hydroxypropyltrimethylammoniumchlorid-Guarkernmehl können noch weitere Konditionierungsmittel vorliegen. Bei diesen kann es sich insbesondere um nichtflüchtige Silikone (Polyorganosiloxane) mit einer Viskosität von 10 000 bis 10⁶ mPa.s in Form von Partikeln mit einem Durchmesser von unter 35 Manometern, vorzugsweise von ungefähr 20 bis ungefähr 25 Nanometern, handeln.

Diese Silikone werden vorzugsweise in Form einer vorgebildeten wäßrigen Dispersion verwendet, die eine Konzentration von ungefähr 20 bis ungefähr 60 Gew.-%, vorzugsweise von ungefähr 30 bis ungefähr 50 Gew.-%, aufweisen kann. Sie können in den Kosmetikformulierungen auch in einer Menge von ungefähr 0,1 bis ungefähr 1 Gew.-%, vorzugsweise von ungefähr 0,5 bis ungefähr 1 Gew.-%, Wirkstoff bezogen auf das Gewicht der Kosmetikformulierung verwendet werden.

Als Silikone können Polydimethylsiloxanöle, phenylierte Silikonöle (Diphenyldimeticone) und aminierte Silikonöle (Amodimeticone) genannt werden.

Schließlich betrifft die Erfindung klare wäßrige Kosmetikformulierungen, die zur Anwendung auf Haare und/oder Haut bestimmt sind und wieder aus- oder abgespült werden sollen, wobei diese Formulierungen bezogen auf ihr Gewicht folgendes enthalten:
ungefähr 8 bis ungefähr 30 Gew.-%, vorzugsweise ungefähr 10 bis ungefähr 20 Gew.-%, mindestens eines nichtionischen, anionischen, amphoteren oder zwitterionischen Tensids, ausgedrückt als Wirkstoff,
ungefähr 0,05 bis ungefähr 0,5 Gew.-%, vorzugsweise ungefähr 0,1 bis ungefähr 0,3 Gew.-%, Hydroxypropyltrimethylammoniumchlorid-Guarkernmehlderivat eines nach dem erfindungsgemäßen Verfahren hergestellten Guarkernmehls,
gegebenenfalls ungefähr 0,1 bis ungefähr 1 Gew.-%, vorzugsweise ungefähr 0,5 bis ungefähr 1 Gew.-%, mindestens eines nichtflüchtigen Silikons in einer wäßrigen. Emulsion, dessen Teilchengröße weniger als 35 Nanometer, vorzugsweise ungefähr 20 bis ungefähr 25 Nanometer, beträgt, ausgedrückt als Wirkstoff,
und mindestens 60 Gew.-% Wasser, vorzugsweise mindestens 75 Gew.-% Wasser.

Die erfindungsgemäßen klaren wäßrigen Kosmetikformulierungen weisen bei 600 Nanometern eine Durchlässigkeit von mindestens 92% auf.

Unter den nichtionischen, anionischen, amphoteren oder zwitterionischen Tensiden, die vorliegen können, können folgende genannt werden:
- anionische Tenside wie
   Alkylsulfate der Formel ROSO₃M, wobei R einen C₁₀-C₂₄-, vorzugsweise C₁₂-C₂₀- und ganz besonders bevorzugt C₁₂-C₁₈-Alkyl- oder -Hydroxyalkylrest darstellt, M ein Wasserstoffatom oder ein wie oben definiertes Kation sowie deren Ethylenoxid (EO)- und/oder Propylenoxid (PO)-Derivate mit durchschnittlich 0,5 bis 6, vorzugsweise 0,5 bis 3, EO- und/oder PO-Einheiten darstellt;
   Salze von gesättigten oder ungesättigten C₈-C₂₄-, vorzugsweise C₁₄-C₂₀-Fettsäuren, C₉-C₂₀-Alkylbenzolsulfonate, primäre oder sekundäre C₈-C₂₂-Alkylsulfonate, Alkylglycerinsulfonate, sulfonierte Polycarbonsäuren wie in GB-A-1 082 179 beschrieben, Paraffinsulfonate, N-Acyl-N-Alkyltaurate, Alkyl- und/oder Alkylether- und/oder Alkylarylether-Phosphorsäureester, Isethionate, Alkylsuccinamate, Alkylsulfosuccinate, Sulfosuccinatmonoester oder -diester, N-Acylsarcosinate, Alkylglycosidsulfate, Alkylpolyethoxycarboxylate; wobei es sich bei dem Kation um ein Alkali- oder Erdalkalimetall (Natrium, Kalium, Lithium, Magnesium), einen gegebenenfalls substituierten Ammoniumrest (Methyl-, Dimethyl-, Trimethyl-, Tetramethylammonium, Dimethylpiperidinium u.a.) oder ein Alkanolaminderivat (Monoethanolamin, Diethanolamin, Triethanolamin u.a.) handelt;
   Alkylsulfonsäureester der Formel R-CH(SO₃M)-COOR', wobei R einen C₈-C₂₀-, vorzugsweise C₁₀-C₁₆-Alkylrest bedeutet, R' einen C₁-C₆-, vorzugsweise C₁-C₃-Alkylrest bedeutet und M ein Alkalimetallkation (Natrium, Kalium, Lithium), gegebenenfalls substituiertes Ammonium (Methyl-, Dimethyl-, Trimethyl-, Tetramethylammonium, Dimethylpiperidinium u.a.) oder ein Alkanolaminderivat (Monoethanolamin, Diethanolamin, Triethanolamin u.a.) darstellt. Ganz besonders bevorzugt seien die Sulfonsäuremethylester mit einem Rest R von C₁₄ bis C₁₆ genannt;
   Alkylamidsulfate der Formel RCONHR'OSO₃M, in der R einen C₂-C₂₂-, vorzugsweise C₆-C₂₀-Alkylrest darstellt, R' einen C₂-C₃-Alkylrest darstellt, M ein Wasserstoffatom oder ein wie oben definiertes Kation sowie deren Ethylenoxid (EO)-und/oder Propylenoxid (PO)-Derivate mit durchschnittlich 0,5 bis 60 EO- und/oder PO-Einheiten darstellt;
   Alkyl- und Dialkylphosphate und -phosphatether;
- nichtionische Tenside wie
   polyalkoxylierte aliphatische qC₈-C₂₂-Alkohole mit 1 bis 25 Alkylenoxideinheiten (Ethylenoxid, Propylenoxid); beispielsweise seien TERGITOL 15-S-9, TERGITOL 24-L-6 NMW, Vertrieb: Union Carbide Corp., NEODOL 45-9, NEODOL 23-65, NEODOL 45-7, NEODOL 45-4, Vertrieb: SHELL CHEMICAL Cy., KYRO EOB, Vertrieb: THE PROCTER & GAMBLE Cy., genannt;
   Glucosamide, Glucamide;
   die in US-A-4 565 647 beschriebenen Alkylpolyglycoside und ihre Polyalkylenoxidderivate;
   polyalkoxylierte (polyethoxylierte, polypropyloxylierte, polybutoxylierte) Alkylphenole, deren Alkylsubstituent C₆-C₁₂-Alkyl ist und die 5 bis 25 Alkylenoxideinheiten enthalten; beispielsweise seien TRITON X-45, X-114, X-100 oder X-102, Vertrieb: ROHM & HAAS Cy, genannt;
   Glycerinamidderivate von N-Alkylaminen (US-A-5,223,179 und FR-A-1 585 966);
   Kondensationsprodukte aus Ethylenoxid oder Propylenoxid mit Propylenglykol, Ethylenglykol und/oder Glycerin, wie die PLURONIC-Reihe, Vertrieb: BASF;
   Kondensationsprodukte aus Ethylenoxid oder Propylenoxid mit Ethylendiamin, wie die TETRONIC-Reihe, Vertrieb: BASF;
   Aminoxide wie C₁₀-C₁₈-Alkyldimethylaminoxide, C₈-C₂₂-Alkoxyethyldihydroxyethylaminoxide;
   C₈-C₂₀-Fettsäureamide;
   ethoxylierte Fettsäuren;
   ethoxylierte Fettsäureamide;
   ethoxylierte Amine;
   ethoxylierte Amidoamine, besonders diejenigen, die sich von den N-Hydroxyethyl-N'-Alkylamidethylendiaminen ableiten;
- amphotere und zwitterionische Tenside wie
   Alkylbetaine, Alkyldimethylbetaine, Alkylamidopropylbetaine, Alkylamidopropyldimethylbetaine, Alkyltrimethylsulfobetaine, Imidazolinderivate wie Alkylamphoacetate, Alkylamphodiacetate, Alkylamphopropionate, Alkylamphodipropionate, Alkylsultaine oder Alkylamidopropylhydroxysultaine, Kondensationsprodukte aus Fettsäuren und Proteinhydrolysaten, amphotere Alkylpolyaminderivate wie AMPHOLIC XL, Vertrieb: RHONE-POULENC, AMPHOLAC 7T/X und AMPHOLAC 7C/X, Vertrieb: BEROL NOBEL.

Diese Tenside werden vorzugsweise aus der Reihe der anionischen Tenside, wie der Alkylsulfate und/oder Alkylsulfatether ausgewählt, vorzugsweise in Kombination mit mindestens einem amphoteren Tensid, wie den Alkylamidopropylbetainen und/oder Alkylamphoacetaten oder -diacetaten, und gegebenenfalls in Kombination mit mindestens einem nichtionischen Tensid, wie den polyalkoxylierten aliphatischen Alkoholen und/oder Glucamiden und/oder Alkylglucosiden.

Ganz besonders bevorzugt umfassen diese Kosmetikformulierungen eine Tensidmischung bestehend aus
ungefähr 5 bis ungefähr 20%, vorzugsweise ungefähr 10 bis ungefähr 15%, mindestens eines aniohischen Tensids, insbesondere eines Alkylsulfats und/oder Alkylethersulfats,
ungefähr 0,1 bis ungefähr 15%, vorzugsweise ungefähr 1 bis ungefähr 5%, mindestens eines amphoteren Tensids, insbesondere Alkylamidopropylbetain und/oder Alkylamphoacetat oder -diacetat und
ungefähr 0 bis ungefähr 5%, vorzugsweise ungefähr 1 bis ungefähr 3%, mindestens eines nichtionischen Tensids, insbesondere eines polyalkoxylierten aliphatischen Alkohols und/oder eines Glucamids und/oder eines Alkylglucosids,
wobei die Prozentsätze Gewichtsprozent Tensidwirkstoff in den Kosmetikformulierungen bedeuten.

Diese klaren wäßrigen Kosmetikformulierungen können außerdem weitere Zusatzstoffe enthalten, die so ausgewählt sind, daß sie die Klarheit dieser Formulierungen nicht verringern.

Die erfindungsgemäßen Kosmetikformulierungen können außerdem auch folgendes enthalten:
- bis zu 10%, vorzugsweise bis zu 5%, Polymerderivate, die auf die Haut eine schützende oder feuchtigkeitsspendende Wirkung, oder eine konditionierende Wirkung, ausüben wie modifizierte Cellulosen (z.B. Hydroxyethylcellulose, Carboxymethylcellulose), oder nichtionische Derivate (z.B. Hydroxypropyl-Guarkernmehl), anionische Derivate (Carboxymethyl-Guarkernmehl) oder nichtionische/anionische Derivatmischungen wie Carboxyhydroxypropyl-Guarkernmehle; es können jedoch auch stattdessen oder zusätzlich synthetische Polymere wie Polyacrylate oder synthetische kationische Polymere, die unter der allgemeinen CTFA-Bezeichnung "Polyquaternium" bekannt sind, zum Beispiel die Polymere MIRAPOL A15 oder MIRAPOL 550 von RHONE-POULENC, oder Polymere, die Frisiereigenschaften verleihen, wie Vinylpyrrolidon-Copolymere, zugegeben werden;
- bis zu 5% Feuchthaltemittel oder Feuchtigkeitsspender, wie insbesondere Glycerin;
- bis zu 5% Calciumkomplexiermittel, wie Citrationen;
- bis zu 1% Sonnenschutzfilter wie Octylmethoxycinnamat (PARSOL LCX von GIVAUDAN);
- bis zu 0,3% Bakterizide wie Triclosan, Chlorphenesin;
- bis zu 1% Konservierungsmittel wie p-Hydroxybenzoesäure-methylester, -ethylester, -propylester und -butylester, Natriumbenzoat, GERMABEN (Handelsname);
- bis zu 0,5% Verdickungsmittel, Gelierungsmittel oder Stabilisatoren wie die von GOODRICH vertriebenen vernetzten Polyacrylate CARBOPOL, Xanthangummi, Succinoglycanderivate;
- Parfums, Färbemittel.

Das aus der vorliegenden Erfindung resultierende Material ist besonders vorteilhaft, da es in Wasser gelöst, Lösungen von hoher Klarheit ergibt. Eine 1 %ige Lösung (0,9 % Trockensubstanz) des mit diesem neuen Verfahren hergestellten reinen Guarkernmehls zeigt eine Viskosität von 6.000 bis 9.000 mPa.s bei 25° C. Eine Transparenz der wässrigen Lösung von bis zu 95 % kann erzielt werden.

Die Viskosität wurde in einem Brookfield RVT-Viskosimeter, die Transparenz der Lösungen in einem Photospektrometer bestimmt.

Die Erfindung wird im folgenden anhand von einigen Beispielen erläutert. Als Ausgangsmaterial für die beschriebenen Beispiele wurden Splits der höchsten Qualität verwendet.

Aus diesen Beispielen ist ersichtlich, dass eine Behandlung der Peripherie der Guarsplits mit einer optimierten Konzentration an Natronlauge in Anwesenheit von geringen Mengen Wasserstoffperoxid bereits zu reinen Guarprodukten führt.

Diese reinen Produkte zeigen Viskositäten von 5000 bis 9000 mPa.s bei 1 %iger Konzentration sowie Klarheiten von mehr als 80 % bei 0,5 %iger Konzentration und 1 cm Lichtweg bei 500 nm, wohingegen herkömmliche Guar-Produkte in wässriger Lösung Lichttransmissionen von 45 bis 48 % zeigen.

Diese Lichtdurchlässigkeit kann bereits ohne Isopropanolbehandlung erreicht werden. Mit Isopropanol werden Lichtdurchlässigkeiten von bis zu 98 % erzielt, da mit Isopropanol Phospholipide entfernt werden, die durch Waschen mit Wasser nicht zu entfernen sind.

Nach diesem ersten Reinigungsschritt weisen die alkalische Splits einen Wassergehalt von 12 bis 15 % auf.

Die Splits werden zweimal mit Wasser in einem Verhältnis von 1:7 und 1:6 während 6 beziehungsweise 8 Minuten gewaschen.
Dabei nehmen die Splits Wasser bis zu 70 % auf und können entweder nach Zugabe von zusätzlichem Wasser bis zu einem Feuchtigkeitsgehalt von 76 bis 78 % gemahlen oder weiter modifiziert werden.
Es ist jedoch möglich, die Splits ohne Waschschritt weiter zu behandeln.

Die gemahlenen Produkte besitzen einen Proteingehalt von 1,0 bis 1,2 % und einen A.I.R.-Anteil von ungefähr 0,8. Die Ausbeute dieser Produkte beträgt, je nach Qualität des Ausgangsmaterials, 75 bis 79 %.

### Beispiel I

370 Guar Splits mit einem Galaktomannangehalt von mindestens 84 % werden z.B. in einem vorgeheizten Sigma-Mischer mit 10 % NaOH (84 ml einer 33 %igen NaOH Lösung) und anschliessend nach einer Minute zusätzlich noch mit 4 ml einer 35 %igen H₂O₂ Lösung, die mit 20 ml Isopropanol verdünnt worden war, behandelt. Die Reaktionstemperatur wird indirekt mittels heissem Wasser von 90° C bis auf 70° C erhöht, und dann 30 Minuten konstant gehalten.

Anschliessend wird ein Teil der anwesenden Lauge durch 21 g 96 %ige H₂SO₄, verdünnt mit 8 g Wasser neutralisiert. Der Mischvorgang wird noch 16 Minuten bei 70°C fortgesetzt. Anschliessend werden schnell 70 g Wasser zugegeben, um die behandelten, störenden Peripheriezellschichten während dem Mischvorgang leichter zu entfernen. Der Mischvorgang wird 30 Minuten bei 70° C durchgeführt.

Das Reaktionsgemisch wird durch ein M20-Sieb gesiebt, wobei
95 g -M20 (H₂O: 16,2 %) und
360 g +M20 (H₂O: 12, 9 %)
erhalten werden.

Die +M20-Fraktion zeigt einen NaOH-Gehalt von 5-6 % und wird zweimal mit kaltem Leitungswasser gewaschen, wobei jeweils 6 Teile Leitungswasser auf 1 Teil der ungewaschenen +M20-Fraktion verwendet werden.

Der Waschvorgang wurde 5 bzw. 6 Minuten unter intensivem Rühren durchgeführt.

Die gewaschenen, gequollenen Splits werden durch einfaches Sieben zurückgewonnen.

(Die in der Literatur und Patentliteratur erwähnten Alkalibehandlungen unter Anwendung von 20 - 32 % NaOH verlangen eine Waschwassermenge von mehr als 40 Teilen Leitungswasser pro 1 Teil ungewaschenen Splits).

916 g gequollene Splits mit einem Wassergehalt von 73,3 % wurden nach der Reinigung mit Wasser zurückgewonnen.

Eine Viskosität von 7'000 mPa.s wurde bei 20 UpM und 25° C gemessen, nachdem die durch einen Aufschlag erhaltene homogene Lösung der gequollenen Splits über Nacht abgekühlt war.

Die 1 %ige wässrige Lösung wurde in einem Haushalts-Mixer bei höchster Stufe bis etwa 90° C hergestellt. Die zu lösende Menge gequollener Splits wurde berechnet auf ein Wassergehalt von 10 %.

Einer Verdünnung dieser 1 %igen Lösung von 1:1 ergibt bis 500 nm in einer 1cm Küvette eine Lichttransmission von 81,3 %.

### N.B.

Werden 40 % weniger NaOH und 40 % weniger H₂SO₄ (unter sonst gleichen Bedingungen) eingesetzt, wird weniger Abrieb (etwa 12 %) und eine signifikant geringere Licht-Transmission von etwa 75 % erhalten.

### Beispiel II

Diese nach Beispiel I erhaltenen Produkte können in ihrer Qualität (Viskosität, Transparenz) signifikant verbessert werden, indem ihr Protein- und A.I.R.-Gehalt weiter gesenkt wird. Produkte, die nicht mit Wasser gewaschen werden, können mit 25 Gewichtsprozent Isopropanol bei 65 bis 70° C ohne Zugabe von zusätzlicher Lauge während 30-60 Minuten behandelt werden, da die Splits noch ungefähr 5-6 % NaOH enthalten. Werden gewaschene Splits verwendet, ist der Zusatz von 2-5 % Lauge, vorzugsweise Natronlauge, erforderlich.

Im Anschluss an die wässrige Isopropanolbehandlung werden die alkalischen Splits gewaschen, durch Zugabe von weiterem Wasser auf den erforderlichen Feuchtigkeitsgehalt gebracht und gemahlen.

Die Transparenz der wässrigen Lösungen solcher Produkte beträgt 93 bis 95 %. Die Viskosität einer 1 % wässrigen Lösung kann je nach Reaktionsbedingung auf 6000 bis 7000 mPa.s eingestellt werden.

Das auf die vorgängig beschriebene Weise gereinigte, als -M20 bezeichnete Produkt ist ein Guarkernmehl mit einer Viskosität von 1000 bis 1500 mPa.s bei 1 %iger Konzentration, das als Basis für signifikant verbesserte Guar-Produkte oder Derivate dienen kann.

Auch die -M20-Fraktion kann auch für Anwendungen aufgearbeitet werden, die alkalische oxidierte Guar-Produkte oder -Derivate verwenden. Ein Anwendungsgebiet wäre zum Beispiel der Polyesterdruck. Durch die Verwendung der - M20-Fraktion erhält das zu bedruckende Material einen weichen Griff, nachdem die verwendeten Dispersionsfarbstoffe bei extrem hohen Temperaturen fixiert worden sind.

### Beispiel III

8522 g Guar-Splits mit einem Galaktomannan-Gehalt von mindestens 84 % werden z.B. in einem indirekt heizbaren Drais-Mischer mit 2580 g einer 33 %igen NaOH-Lösung, die auf 74° C vorgeheizt worden ist, in Kontakt gebracht.

Nach 3 Minuten werden 230 g einer 14 %igen H₂O₂-Lösung langsam zugesetzt.

Die Temperatur des heterogenen Reaktionsgemisches wird bis auf 70° C erhöht und 30 Minuten aufrecht gehalten.

Anschliessend wird das Reaktionsgemisch zur besseren Kontrolle der partiellen Neutralisation mit 668 g einer 69,5 %igen Schwefelsäure bis auf 55° C herunter gekühlt.

Die Reaktion wird 30 Minuten bei 70° C fortgesetzt. Die behandelten Guar-Splits werden anschliessend mit kaltem Leitungswasser 5 Minuten gewaschen, wobei 12 Teile Leitungswasser auf 1 Teil ungewaschener Splits angewandt werden. Bei der Reaktion in erwähntem Mischer entsteht praktisch kein Abrieb, so dass auf einem Siebvorgang vor dem Waschen verzichtet werden kann.

Die gewaschenen Splits haben einen Wassergehalt von 72 % und werden in üblicher Weise in einem heissen Luftstrom in einer Hammermühle feiner als M150 gemahlen.

Das gemahlene Produkt enthält noch etwa 2 % NaOH und zeigt eine Viskosität von 4200 mPa.s bei einer Konzentration von 1 %, basierend auf 10 % Wasser bei pH von 6,8.

Die Klarheit einer 0,5 %igen Lösung, die wie beschrieben, gemessen wurde, beträgt 82,4 %.

### Beispiel IV

25 kg Guar Splits mit gleicher, wie in den Beispielen I und II verwendeter Qualität, werden in einem Sigma-Mischer mit 9 % NaOH in Anwesenheit von 0,67 kg einer 14,1 %igen H₂O₂-Lösung, während einer halben Stunde bei 70° C behandelt. Es wurde eine 33 %ige NaOH-Lösung eingesetzt.

Anschliessend wurde während 15 Minuten bei 70° C und offenem Reaktor die Peripheriezellschichten abgerieben. 6,5 gewichtsprozentige -M20 konnten entfernt werden.

Die +M20 Fraktion wurde, wie beschrieben, mit Leitungswasser gewaschen, wobei ein Produkt mit einer Viskosität von 5700 mPa.s (Konzentration 1 %) und einer Klarheit (0,5 %) von 81,3 % erhalten wurde.

Die noch anhaftenden behandelten Peripheriezellschichten können mechanisch durch eine intensive Reibung der +M20 Fraktion z.B. in einer Haushalts-Kaffeemühle, bei niedrigster Mahlgeschwindigkeit abgerieben werden.

Durch wiederholtes "Mahlen" konnten noch zusätzlich etwa 12 % der Peripheriezellschichten entfernt werden, so dass insgesamt etwa 18-19 % der +M20-Fraktion zurückgewonnen wurden.

Diese Periperiezellschichten lassen sich ebenfalls in wässrigen Alkoholen unter intensivem Rühren abreiben.

Wird z.B. 1 Teil des +M20-Fraktion 5 Minuten in 1,2 Teilen 35 gewichtsprozentigem Methylalkohol intensiv gerührt und dieser Vorgang 3 mal wiederholt, so können 13,5 % der +M20-Fraktion zurückgewonnen werden, so dass insgesamt 20 % dieser Fraktion erhalten bleiben.

### Beispiel V

### Kationische Derivatisierung

Das Produkt aus Beispiel III wurde gemahlen und 400 g dieses gemahlenen Produktes wurden in 1600 g eines 25 gewichtsprozentigen wässrigen Isopropanollösung resuspendiert. 100 ml einer 30 gewichtsprozentigen NaOH-Lösung wurden zugegeben. Die Suspension wurde in einer Stickstoffatmosphäre während 30 Minuten unter ständigem Rühren auf 70° C erhitzt. Diese Reaktionstemperatur wurde 1 Stunde aufrechterhalten, dann wurde die Suspension auf 55° C abgekühlt. Das Rühren wurde unterbrochen und nach dem Absetzen des Reaktionsprodukts wurde der Überstand abgegossen.

Das Reaktionsprodukt wurde mit 1000 ml einer 50 Gewichtsprozentigen Isopropanollösung gewaschen und anschliessend mit 10 ml Eisessig behandelt, um eine stöchiometrische Menge NaOH zu neutralisieren.

Der Überstand wurde nach Absetzen des Reaktionsprodukts abgegossen. 7,8 g NaOH wurden auf diese Weise entfernt.

225 g einer 40 gewichtsprozentigen 2,3-Epoxypropyltrimethylammoniumchloriodlösung wurde zugegeben und während 1 Stunde bei 30° C in das alkalisch behandelte Produkt eindringen gelassen. Danach wurden 1100 ml eines 85 gewichtsprozentigen Isopropanollösung als Suspensionsmedium verwendet. Die Stickstoffatmosphäre wurde wieder hergestellt und die Suspension auf 65° C erhitzt. Diese Temperatur wurde 40 Minuten konstant gehalten.

Anschliessend wurde die Stickstoffzufuhr unterbrochen, um das Reaktionsprodukt mit Luftsauerstoff in Kontakt zu bringen und so die endgültige Viskosität des Kationischen Guarproduktes einzustellen.

Die Reaktion wurde 45 Minuten bei 65° C durchgeführt. Das Produkt wurde anschliessend mit 800 ml einer 85 Gewichtsprozentigeh Isopropanollösung dann mit 1000 ml dergleichen Lösung gewaschen. Während dem Waschvorgang wurden 25 ml 99 gewichtsprozentiger Eisessig zugegeben, wodurch die Lauge neutralisiert wurde.

Das Produkt wurde durch Filtration zurückgewonnen und mit heisser Luft bei 70° C getrocknet.

Dieses Kationische Guar wies eine Viskosität bei 1 %igen Konzentration in Wasser von 880 mPa.s (auf 10 % Feuchtigkeit basierend) und eine Lichttransmission (Klarheit) von 94,2 % auf.

### Beispiel VI

### Carboxymethylierung

350g einer +M20-Fraktion mit einem Feuchtigkeitsgehalt von 7,5 % wurden mit 100ml kaltem Wasser befeuchtet. Nach 15 Minuten wurde der grösste Teil der Monochloracetat-Na-Lösung, bestehend aus 79 g Monochloracetat-Na und 184 g Wasser während 24 Minuten unter Rühren langsam zugegeben. Die übrigen 100 ml dieser Lösung wurden während 5 Minuten schnell zugegeben. Die Zugabe der Reagenzien erfolgte bei Raumtemperatur.

Nach weiteren 16 Minuten Inkubation unter Rühren wurde die Temperatur auf 50°C erhöht. Nun wurden 27 g NaOH-Plätzchen zugegeben. Aufgrund der exothermen Reaktion der verwendeten NaOH-Plätzchen stieg die Temperatur schnell auf 65-66°C an und wurde 36 Minuten beibehalten.

Das Reaktionsprodukt wurde aus dem Reaktor entfernt und zweimal mit Wasser in einem Verhältnis von 1:6 gewaschen. Aufgrund der sehr schnellen Wasseraufnahme der gereinigten, nicht mit Borax behandelten Carboxymethyl-Splits, wurde deren Verweildauer in Wasser jeweils auf 2 Minuten beschränkt. Das Gewicht der stark gequollenen Splits betrug 2640 g. Daher war ein Entwässerungsschritt mit Isopropanol (1500g wurden verwendet), notwendig.

3261 g des Filtrats wurden zurückgewonnen. Das Gewicht der mit Alkohol befeuchteten, carboxymethylierten Splits betrug 879 g mit einer flüchtigen Menge von 66,8 %.

Da keine Vorsichtsmassnahmen während der Reaktion durch eine inerte Stickstoffhaube getroffen wurde, waren die Splits während der gesamten Reaktionszeit mit Luft in Kontakt, was eine beträchtliche Depolymerisation verursachte.

Die Viskosität einer 1 %igen-Lösung der carboxylierten Splits betrug 1720 mPa.s bei einer Klarheit von 92,7 %. Diese Werte wurden bei einer Temperatur von 25° C erhalten.

### Beispiel VII

400 g der bevorzugten Guarsplitqualität mit einem Galaktomannahgehalt von mindestens 84 % wurden in einem vorgeheizten Sigma-Mischer zuerst mit 4 ml 35 % H₂O₂, verdünnt, mit 21 ml entmineralisiertem Wasser während 9 Minuten behandelt, wonach 40 g NaoH, gelöst in 61 ml entmineralisiertem Wasser heiss zugesetzt wurden.

Innerhalb von 3 Minuten erreichte das Reaktionsgemisch die erwünschte Temperatur von 68° C. Die Reaktionstemperatur von 68° - 72° C wurde 20 Minuten aufrecht gehalten. Anschliessend wurde dem Reaktionsgemisch das meiste Wasser durch Ventilation und indirekter Erhitzung während 23 Minuten entzogen.

Das Reaktionsgemisch wurde über M20 gesiebt, wobei 30g -M20 und 437 g +M20 (H₂O-Gehalt 10,1 %) erhalten wurden. Die +M20-Fraktion wurde zweimal mit Wasser gewaschen, wobei jeweils 6 Teile Leitungswasser auf 1 Teil der ungewaschenen +M20-Fraktion verwendet wurden. Die jeweiligen Waschzeiten waren 4 und 5 Minuten.

Die gewaschenen, gequollenen Splits wurden durch einfaches Sieben zurückgewonnen. 892 g gequollene Splits wurden erhalten, die einen Wassergehalt von 67,8 % aufwiesen. Diese Splits wurden wie üblich in Lösung gebracht und diese Lösung erzeugte eine Viskosität von 2300 mPa.s und eine Lichttransmission von 86,4 % (gemessen nach Beschreibung in Beispiel I).

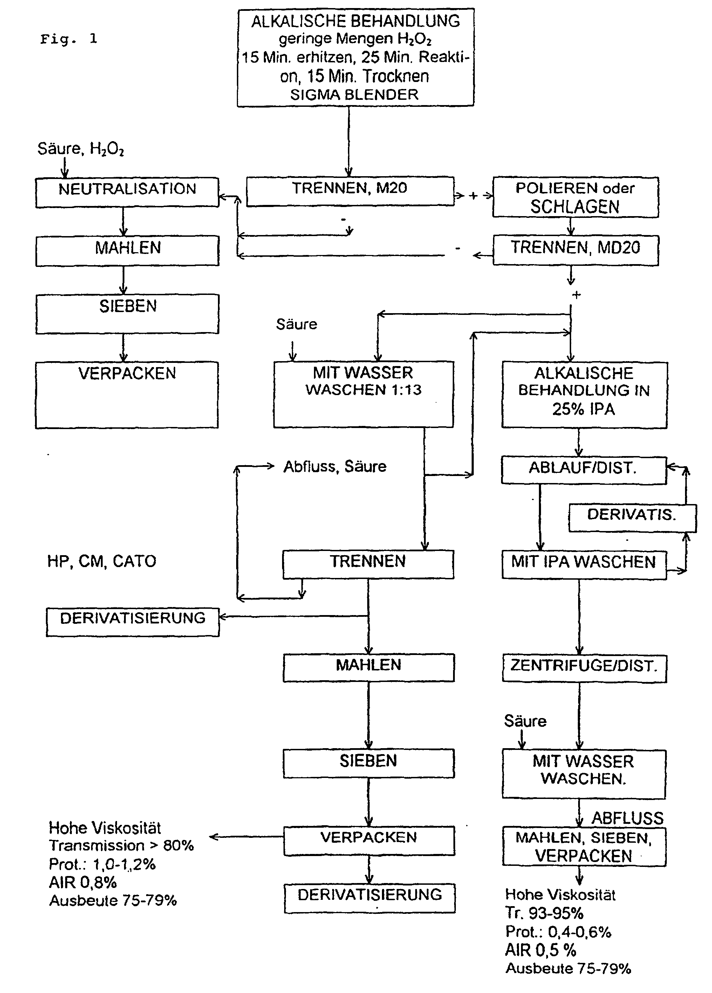

Fig. 1: Das Flussdiagramm zeigt die erfindungsgemässe Behandlung der Splits mit Lauge und Wasserstoffperoxid und die sich an die alkalische Behandlung anschliessenden Weiterbearbeitungsmöglichkeiten.

### Beispiel VIII

### Klares Konditionierungsshampoo

Ein Konditionierungsshampoo der folgenden Zusammensetzung wird nach dem Fachmann bekannten Verfahren hergestellt:

| Bestandteile | g (Wirkstoff) / 100 g |
|---|---|
| Natriumlaureth-2-sulfat (wäßrige Lösung von sulfatiertem ethoxyliertem Laurylalkohol mit 28% Wirkstoff) | 7,5 |
| Cocamidopropylbetain (wäßrige | 2,5 |
| Lösung mit 25% Wirkstoff) | |
| Hydroxypropyltrimethylammonium-chlorid-Guarkernmehl von Beispiel 5 (100% WirkStoff) | 0,3 |
| Wasser | ad 100 |

Diese Formulierung wird durch Zugabe von Natriumchlorid auf eine Viskosität von 3000 mPa.s eingestellt.

Die bei 600 nm gemessene Durchlässigkeit beträgt 97%.

### Beispiel IX

### Klares Duschgel

Ein klares Duschgel der folgenden Zusammensetzung wird nach dem Fachmann bekannten Verfahren hergestellt (Zugabe der Mikroemulsion von aminomodifiziertem Silikon zum Rest der Mischung):

| Bestandteile | g (Wirkstoff) / 100 g |
|---|---|
| Natriumlaureth-2-sulfat (wäßrige Lösung von sulfatiertem ethoxyliertem Laurylalkohol mit 28% Wirkstoff) | 8 |
| Cocamidopropylbetain (wäßrige Lösung mit 25% Wirkstoff) | 4 |
| Hydroxypropyltrimethylammonium-chlorid-Guarkernmehl von Beispiel 5 (100% Wirkstoff) | 0,2 |
| MIRASIL ADME (RHODIA CHIMIE) (wäßrige Amodimeticon-Mikroemulsion mit 30% Wirkstoff und einer Teilchengröße von 25 nm) | 0,3 |
| Wasser | ad 100 |

Diese Formulierung wird durch Zugabe von Natriumchlorid auf eine Viskosität von 2500 mPa.s eingestellt.

Die bei 600 nm gemessene Durchlässigkeit beträgt 96%.

## Patentansprüche

1. Verfahren zur Herstellung von reinem Guarkernmehl, **dadurch gekennzeichnet, dass** es die folgenden Schritte umfasst:
(a) Behandeln der Guar-Splits mit einer alkalischen Lösung in Anwesenheit von 0,175 bis 0,35 Gewichtsprozent, auf die Gesamtmasse bezogen, einer Wasserstoffperoxidlösung bei 65-70°C;
(b) teilweise Neutralisation der alkalischen Splits mit einer organischen oder anorganischen Säure;
(c) mechanisches Entfernen der peripheren Zellschichten der Guar-Splits;
(d) Behandeln der Splits mit einer wässrigen Alkohollösung; und
(e) Vermahlen der Splits zu Mehl.

2. Verfahren nach Anspruch 1, **dadurch gekennzeichnet, dass** die in Schritt (a) verwendete alkalische Lösung Natronlauge ist, vorzugsweise in einer Konzentration von 25 bis 50 Gewichtsprozent, am meisten bevorzugt in einer Konzentration von 33 Gewichtsprozent und in einer Menge von 6-10% basierend auf dem Ansgangsmaterial. Phosphorsäure oder eine geeignete organische Säure, vorzugsweise Essigsäure ist.

3. Verfahren nach Anspruch 1 oder 2, **dadurch gekennzeichnet, dass** die in Schritt (a) verwendete Menge Wasserstoffperoxid vorzugsweise 0,175 Gewichtsprozent umfasst, wobei eine Wasserstoffperoxidlösung von 35 Gewichtsprozent verwendet wird.

4. Verfahren gemäss einem der vorstehenden Ansprüche, **dadurch gekennzeichnet, dass** die Säure in Schritt (b) Schwefelsäure,

5. Verfahren nach Anspruch 4, **dadurch gekennzeichnet, dass** die in Schritt (b) verwendete Schwefelsäure in einer Konzentration von 60 bis 80 Gewichtsprozent, vorzugsweise von 70 Gewichtsprozent verwendet wird.

6. Verfahren nach Anspruch 4, **dadurch gekennzeichnet, dass** die in Schritt (b) verwendete Phosphorsäure in einer Konzentration von 60 bis 85 Gewichtsprozent, vorzugsweise von 75 Gewichtsprozent verwendet wird.

7. Verfahren nach einem der vorstehenden Ansprüche, **dadurch gekennzeichnet, dass** die in Schritt (d) verwendete Alkohollösung eine wässrige Lösung von Ethanol, Methanol oder vorzugsweise Isopropanol ist.

8. Verfahren nach Anspruch 7, **dadurch gekennzeichnet, dass** die wässrige Alkohollösung vorzugsweise eine 20 bis 40 gewichtsprozentige, am meisten bevorzugt eine 25 gewichtsprozentige Isopropanollösung ist.

9. Verfahren nach Anspruch 7 oder 8, **dadurch gekennzeichnet, dass** die Splits nach Entfernen der peripheren Zellschichten in Schritt (c) und vor der Behandlung mit einer wässrigen Isopropanollösung in Anwesenheit einer Lauge, vorzugsweise Natronlauge, bei 60 bis 70°C in Schritt (d) zweimal mit Wasser gewaschen werden.

10. Verfahren zur Herstellung von Guarkernmehlderivaten, wobei im Anschluss an das Verfahren nach einem der Ansprüche 1-9 das reine Guarkernmehl einer Derivatisierung mittels einer Carboxymethylierung, Hydroxypropylierung oder Kationisierung oder Kombinationen davor unterzogen wird.

11. Verfahren zur Herstellung von Guarkernmehlderivaten, wobei im Anschluss an das Verfahren nach einem der Ansprüche 1-9 das erhaltene reine Guarkernmehl mit 2,3-Epoxytrimethylammoniumchlorid als ein kationisches Veretherungsagenz unter basischen Bedingungen und unter einer inerten Gasatmosphäre verethert wird, gefolgt von einer Reinigung und einer Trennung, um Hydroxypropyltrimethylammoniumchlorid-Guarkemmehl zu ergeben.

12. Verfahren gemäss Anspruch 11, worin die Veretherung in zwei primären Schritten durchgeführt wird:
einem initialen Schritt, in dem das kationische verethernde Agenz in das Guarkernmehl unter alkalischen Bedingungen und in einer inerten Gasatmosphäre bei einer Temperatur von 20 bis 55°C, vorzugsweise von 30 bis 50°C diffundiert wird und anschliessend in einer wässrigen Alkohollösung, insbesondere einer 25 bis 70 Gewichtsprozent Isopropanol enthaltenden Wasser-Isopropanollösung suspendiert wird, und
einem zweiten Schritt, in dem die eigentliche Veretherung unter einer inerten Gasatmosphäre bei einer Temperatur von 50°C bis 70°C, am meisten bevorzugt bei einer Temperatur von 60°C bis 65°C durchgeführt wird, um Hydroxypropyltrimethylammoniumchlorid-Guarkernmehl zu ergeben.

13. Verwendung des nach einem der Ansprüche 11 oder 12 hergestellten Guarkernmehls, zur Verwendung als Verdickungsmittel für Lebensmittel, für Kosmetika, für pharmazeutische Produkte, für Farben und Lacke, für Textilfarben, für die Erdölgewinnung und für explosiv Stoffe.

14. Verfahren nach einem der Ansprüche 1 bis 13, **dadurch gekennzeichnet, dass** die Wasserstoffperoxidlösung 14 bis 35 Gewichtsprozent aufweist.

## Claims

1. Process for producing pure guar gum meal, **characterized in that** it comprises the following steps:
(a) treating guar splits with an alkaline solution in presence of a hydrogen peroxide solution of 0,17 to 0,35 per cent by weight of the total mass at 65 to 70 ° Celsius,
(b) partially neutralizing the alkaline splits with an organic or inorganic acid,
(c) mechanical removing of the peripheral cell layers of the guar splits,
(d) treating the splits with an aqueous alcohol solution, and
(e) grinding the splits into meal.

2. Process according to claim 1, **characterized in that** the alkaline solution being used in step (a) is sodium hydroxide solution, preferably at a concentration of 25 to 50 per cent by weight, most preferred at a concentration of 33 per cent by weight and within a proportion of 6 to 10 per cent of the original matter.

3. Process according to claim 1 or 2, **characterized in that** the amount of hydrogen peroxide being used in step (a) is preferably 0,175 per cent by weight, wherein a hydrogen peroxide solution of 35 per cent of weight is used.

4. Process according to one of the preceding claims, **characterized in that** the acid being used in step (b) is sulfuric acid, phosphoric acid or an appropriate organic acid, preferably acetic acid.

5. Process according to claim 4, **characterized in that** the sulfuric acid being used in step (b) has a concentration between 60 and 80 per cent by weight, preferably 70 per cent by weight.

6. Process according to claim 4, **characterized in that** the phosphoric acid being used in step (b) has a concentration between 60 and 85 per cent by weight, preferably 75 per cent by weight.

7. Process according to one of the preceding claims, **characterized in that** the alcohol solution being used in step (d) is an aqueous solution of ethanol, methanol or preferably isopropanol.

8. Process according to claim 7, **characterized in that** the aqueous alcohol solution is a isopropanol solution at a concentration of preferably between 20 and 40 per cent by weight, most preferred at a concentration of 25 per cent by weight.

9. Process according to claim 7 or 8, **characterized in that** after removal of the peripheral cell layers in step (c) and before the treatment with an aqueous isopropanol solution in presence of an alkaline solution, preferably a sodium hydroxide solution, the splits are washed twice with water at 60 to 70 **°** Celsius in step (d).

10. Process for producing guar gum meal derivatives, wherein following to the process according to one of claims 1 to 9 the pure guar gum meal is subjected to a derivatization using carboxy methylation, hydroxy propylation or cationizing or combinations thereof.

11. Process for producing guar gum meal derivatives, wherein following to the process according to one of claims 1 to 9 the obtained pure guar gum meal is esterified with 2,3 epoxy trimethyl ammonium chloride as cationic esterifying agent under alkaline conditions and within an inert gas atmosphere, followed by a cleaning and a separation to obtain hydroxy propyl trimethyl ammonium chloride guar gum meal.

12. Process according to claim 11, wherein esterifying is conducted in two primary steps:
- an initial step, within which the cationic esterifying agent is diffused into the guar gum meal under alkaline conditions and within an inert gas atmosphere at a temperature between 20 and 55 ° Celsius, preferably between 30 and 50 ° Celsius, and, subsequently, is suspended in an aqueous alcohol solution, especially in a water-isopropanol solution comprising isopropanol at a concentration of 25 to 70 per cent by weight, and
- a second step, wherein the actual esterifying is conducted within an inert gas atmosphere at a temperature between 50 and 70 ° Celsius, most preferred at a temperature between 60 and 65 ° Celsius, to obtain hydroxy propyl trimethyl ammonium chloride guar gum meal.

13. Use of the guar gum meal produced according to one of claims 11 or 12, as thickening agent in foodstuffs, in cosmetics, in pharmaceutical products, in colours and in finishers, in textile colours, and in the oil production and in explosives.

14. Process according to one of claims 1 to 13, **characterized in that** the hydrogen peroxide solution comprises 14 to 35 per cent by weight.

## Revendications

1. Procédé destiné à la fabrication de farine de graines de guar pure, **caractérisé en ce qu'**il comprend les étapes suivantes:
(a) traitement d'éclats de guar avec une solution alcaline aqueuse en présence d'une solution de peroxyde d'hydrogène de 0,17 to 0,35 % en poids de la masse totale entre 65 et 70 ° Celsius,
(b) neutralisation partielle des éclats alcalins avec un acide organique ou anorganique,
(c) enlèvement mécanique des couches de cellules périphériques des éclats de guar,
(d) traitement des éclats avec une solution alcoolisée aqueuse, et
(e) moulage les éclats en farine.

2. Procédé selon la revendication 1, **caractérisé en ce que** la solution alcaline aqueuse de la phase (a) est de la soude caustique, préférablement d'une concentration de 25 à 50 % en poids, le plus préférablement d'une concentration de 33 % en poids et avec une proportion de 6 à 10 % du matériel d'origine.

3. Procédé selon la revendication 1 or 2, **caractérisé en ce que** la proportion de peroxyde d'hydrogène de la phase (a) est préférablement 0,175 % en poids, où une solution de peroxyde d'hydrogène de 35 % en poids est utilisée.

4. Procédé selon l'une quelconque des revendications précédentes, **caractérisé en ce que** l'acide de la phase (b) est de l'acide sulfurique, de l'acide phosphorique ou un acide organique approprié, préférablement de l'acide acétique.

5. Procédé selon la revendication 4, **caractérisé en ce que** l'acide sulfurique de la phase (b) a une concentration entre 60 et 80 % en poids, préférablement 70 % en poids.

6. Procédé selon la revendication 4, **caractérisé en ce que** l'acide phosphorique de la phase (b) a une concentration entre 60 et 85 % en poids, préférablement 75 % en poids.

7. Procédé selon l'une quelconque des revendications précédentes, **caractérisé en ce que** la solution alcoolisée de la phase (d) est une solution aqueuse d'éthanol, de méthanol or préférablement d'isopropanol.

8. Procédé selon la revendication 7, **caractérisé en ce que** la solution alcoolisée aqueuse est une solution d'isopropanol avec une concentration de préférablement entre 20 et 40 % en poids, le plus préférablement avec une concentration de 25 % en poids.

9. Procédé selon la revendication 7 or 8, **caractérisé en ce qu'**après enlèvement des couches de cellules périphériques dans la phase (c) et avant le traitement avec une solution aqueuse d'isopropanol en présence d'une solution alcaline aqueuse, préférablement de la soude caustique, les éclats sont lavés deux fois avec de l'eau à une température de 60 à 70 ° Celsius dans la phase (d).

10. Procédé pour la production de dérivés de farine de graines de guar, où suivant le procédé selon l'une quelconque des revendications précédentes 1 à 9, la farine de graines de guar pure est sujet à une derivatisation avec une méthylation de carboxyle, de la propylation de hydroxyle ou de la cationisation ou des combinaisons de ceux-ci.

11. Procédé pour la production de dérivés de farine de graines de guar, où suivant le procédé selon l'une quelconque des revendications précédentes 1 à 9, la farine de graines de guar pure obtenue est estérifiée avec du 2,3-epoxy-triméthyl chlorure d'ammonium en tant qu'agent cationique estérifiant sous des conditions alcalines et dans une atmosphère de gaz inerte, suivie par un nettoyage et une séparation pour obtenir hydroxyle-propyle-triméthyl chlorure d'ammonium farine de graines de guar.

12. Procédé selon la revendication 11, où l'estérification est conduite dans deux phase primaires:
- une phase initiale, où l'agent cationique estérifiant est diffusé dans la farine de graines de guar sous des conditions alcalines et dans une atmosphère de gaz inerte avec une température entre 20 et 55 ° Celsius, préférablement entre 30 et 50 ° Celsius, et, ensuite, est suspendu dans une solution alcoolisée aqueuse, en particulier dans une solution d'eau avec de l'isopropanol comprenant de l'isopropanol avec une concentration de 25 à 70 % en poids, et
- une phase secondaire, où la véritable estérification est conduite dans une atmosphère de gaz inerte à une température entre 50 et 70 ° Celsius, le plus préférablement à une température entre 60 et 65 ° Celsius, pour obtenir de la farine de graines de guar à l'ammonium chlorure hydroxyle propyle triméthyle.

13. Utilisation de la farine de graines de guar produite selon l'une quelconque des revendications précédentes 11 or 12, en tant qu'agent d'épaississant dans les aliments, les cosmétiques, les produits pharmaceutiques, les couleurs et les laques, les couleurs textiles, et la production de pétrole et les explosives.

14. Procédé selon l' une quelconque des revendications précédentes 1 to 13, **caractérisé en ce que** la solution de peroxyde d'hydrogène comprend 14 à 35 % en poids.
